Europäisches Patentamt

(19) European Patent Office

Office européen des brevets

(11) Publication number: **0 233 048 B1**

## (12) EUROPEAN PATENT SPECIFICATION

(45) Date of publication of patent specification: **02.09.92** (51) Int. Cl.⁵: **G01N 33/569**, G01N 33/543

(21) Application number: **87300968.2**

(22) Date of filing: **04.02.87**

The file contains technical information submitted
after the application was filed and not included in
this specification

(54) **Method of detecting urinary tract infection or inflammation.**

(30) Priority: **05.02.86 GB 8602815**
**03.12.86 GB 8628899**

(43) Date of publication of application:
**19.08.87 Bulletin 87/34**

(45) Publication of the grant of the patent:
**02.09.92 Bulletin 92/36**

(84) Designated Contracting States:
**AT BE CH DE ES FR GB GR IT LI LU NL SE**

(56) References cited:
**EP-A- 0 015 473**
**EP-A- 0 153 477**

**ACTA. PATH. MICROBIOL. SCAND.,** section
C, vol. 87, no. 1, 1979, DK;
A.SOHL-AKERLUND et al., pp. 29-36

**CHEMICAL ABSTRACTS,** vol. 82, no. 9, 03
March 1975, Columbus, OH (US); L.HANSON
et al., p. 394, no. 55476h

**CHEMICAL ABSTRACTS,** vol. 75, no. 1, 05
July 1971, Columbus, OH (US); W.L.HAND et
al., p. 230, no. 2768d

**CHEMICAL ABSTRACTS,** vol. 77, no. 1, 03
July 1972, Columbus, OH (US); W.L.HAND et
al., p. 347, no. 3681y

(73) Proprietor: **University of Dundee**
**Tower Building**
**Dundee DD1 4HN, Scotland(GB)**

(72) Inventor: **Mckenzie, Hamish**
**38 Gotterstone Drive**
**Dundee Scotland(GB)**

(74) Representative: **Pattullo, Norman et al**
**Ian G. Murgitroyd and Company Mitchell**
**House 333 Bath Street**
**Glasgow G2 4ER Scotland(GB)**

Note: Within nine months from the publication of the mention of the grant of the European patent, any person
may give notice to the European Patent Office of opposition to the European patent granted. Notice of opposition
shall be filed in a written reasoned statement. It shall not be deemed to have been filed until the opposition fee
has been paid (Art. 99(1) European patent convention).

**Description**

This invention relates to a method of detecting urinary tract infection or inflammation.

Screening by culture for urinary tract infection or inflammation is one of the most common standard tests performed, being a routine procedure in patients with signs and symptoms of urinary tract infection attending general practitioners. Urine culture is also performed as a screening test in certain patients whether symptoms are displayed or not, in ante-natal clinics, renal clinics and the like. The results of such tests at the present time are indefinite, as the tests produce only a 25-50% positivity rate in clearly symptomatic patients; thus up to 75% of tests performed on symptomatic patients produce a negative result. This leads to considerable uncertainty in evaluating the test results, since a negative finding can indicate either (a) no infection or (b) undetected infection due to the test limitations.

It appears likely that the limited effectiveness of the standard test arises from the occasional non-appearance of infecting organisms in the urine of infected patients or the inability of the organisms present to grow under the conditions used in the standard test. A further disadvantage of the standard test is that the results are not available on the day the sample is taken, since in general at least 16 hours incubation is required for the growth of infecting organisms. Finally, the standard culture test often results in the growth of organisms which are contaminants and not the cause of infection. It is not possible to distinguish readily between these two possibilities in many samples with the standard culture method.

Antibody is a complex protein produced in response to an infecting organism, and it is commonly measured in blood as a means of diagnosing infection.

It has been suggested in Journal of Antimicrobial Chemotherapy (1984) 13, 95-99 to try to detect in the urine of infected individuals organisms which are combined with antibody, but this is dependent on the presence of organisms in the urine, and therefore only applies to samples which are culture positive in the standard test.

It has also been proposed in Acta Path. Microbiol. Scand. Sec. C 87, 29-36, 1979 to combine free antibody in a urine sample with antigen prepared from the organism which has already been isolated from that urine by the standard culture test. Antibody was thus demonstrated to be present in the urine of symptomatic patients with positive urine culture tests, and was shown to be produced at the local site of infection before such antibodies were detected in serum. It was proposed that such antibody may be present in the urine of patients with infection of the upper urinary tract, and that these patients might therefore be distinguished from those with infection of the lower tract. The antibody measured was specific for O antigen prepared in each case from the infecting strain of Escherichia coli. This organism is the commonest cause of urinary tract infection, and many different strains are recognised on the basis of their different antigenic composition. The O antigens are thought to be of particular importance in stimulating the host antibody response, and it is therefore considered that antibody in each patient will be detected only by the use of the O antigen from that patient's infecting strain of organism. Such tests can only be performed therefore on urine samples which are positive in the standard culture test, since a source of appropriate antigen must be obtained, or the O antigen of the infecting strain identified.

Ratner et al described in J. Infec. Dis. (1981) 143, 404-412 the measurement of a urinary antibody to a sonicated antigenic extract of the patient's own isolate of E. Coli by radio-immunoassay. In this case each urine sample was tested against its own corresponding antigen, and only culture-positive samples were examined. Normal urine samples were tested against some of these preparations to give an estimate of background. The results of antibody measurement were assessed for their ability to discriminate between upper and lower tract infection.

In J. Immunoassay (1985) 6, 23-43 an ELISA test was used to measure separately serum antibody to four different strains of E. Coli and of Pseudomonas in normal controls. There was no suggestion that the method could be applied to urine or that mixtures of organisms might be useful.

Jodal reported in Acta Pediatr. Sand. (1975) 64, 96-104 three indirect haemagglutination tests which measured antibody in serum to the patient's own E. Coli 0 antigen, to a mixture of eight 0 antigens and to a mixture of sixty-eight 0 antigens. It was intended by using a mixture of O antigens to detect antibody to a wide range of serotypes of E. coli and therefore cover most possible infections. However there are more than one hundred and fifty O antigens known, and it was found impractical to include them all. The mixture comprising sixty-eight different antigens provided a very insensitive test, since each individual antigen was considerably diluted and was therefore unable to bind an appreciable amount of antibody. The detection of serum antibody by this test was again considered to be of potential value in differentiating between upper and lower urinary tract infection.

Acta Paediatrica Scand. (1967) 56, 637-650 describes the use of a haemagglutination test to measure serum antibody to a mixture of eight 0 antigens from E. Coli as an alternative to using the patient's own strain every time. This was found to be positive in 24 out of 29 patients with positive cultures, and no suggestion was made that a test could be made effectively for diagnosis in culture negative samples, or that it could be used in infections caused by organisms other than those included in the antigen mixture.

According to the present invention there is provided a method of detecting urinary tract infection or inflammation comprising providing a urine sample from a patient, adding to said urine sample a mixture of antigens prepared from a mixture of organisms which are known to occur in urinary tract infection by killing said organisms without substantial reduction of the antigen content of the organisms, said organisms being obtained independently of said patient's urine, and detecting the presence or absence of a combination of antibody from said urine sample with antigen from said mixture of antigens.

Further according to the present invention there is provided a method of detecting urinary tract infection or inflammation wherein the presence or absence of said combination of antibody and antigen is detected by washing said mixture of antigens after addition of said urine sample and adding to the washed mixture a labelled reagent which is known to combine with antibody which combines with said antigens, washing excess of said reagent from said mixture and thereafter detecting the presence or absence of said label on the mixture.

The mixture of antigens is derived from organisms known to be common as the cause of urinary tract infection, and such organisms may be Gram positive or Gram negative. Organisms are included in a killed undisrupted form or after treatment by heating or sonication, to provide antigens for the mixture. An antigen mixture representative of Gram negative organisms is derived from members of the family Enterobacteriaceae, and/or from the genus Pseudomonas. Especially effective is Escherichia coli. Further mixtures are representative of different types of Gram positive infections. One such mixture would represent the genus Staphylococcus, strains of Staphylococcus saprophyticus being especially effective. Similarly a further mixture would represent the genus Streptococcus, strains of Streptococcus faecalis being especially effective. The antigenic mixtures described may be used in combination to diagnose infection or may be used separately to attempt to identify to which group the infecting organism belongs, i.e. Gram negative, Staphylococcus or Streptococcus.

When urinary tract infection by Escherichia coli and other Gram negative bacilli is to be detected, antigens from a mixture of about six organisms have been found to be effective. Other mixtures of antigens or organisms may be suitably representative of other types of infecting organisms, e.g., Gram positive organisms such as Staphylococci and Streptococci. Suitable organisms in a mixture for Gram negative bacilli are:

Escherichia coli

Klebsiella species

Proteus mirabilis

Citrobacter freundii

The mixture of antigens, or of organisms providing the antigens, is preferably immobilised on a solid support, for example on a plastics substrate in the form of strips, beads, tubes or wells, or on Sepharose or Sephadex, and is generally incubated on the plastics at alkaline pH, while it forms a covalent linkage with Sephadex.

Although it has not yet been tested, it is possible that the method may be made more rapid and sensitive if the organisms or purified preparation of bacterial antigens are immobilised on the surface of and/or within the pore structure of a porous membrane, preferably one of high internal surface area and made of a material capable of immobilising large quantities of bacterial antigen. Samples of biological fluids can then be poured, forced or sucked through the membrane. Equilibration of antibodies with immobilised antigen may proceed rapidly because there is no requirement for antibody molecules to diffuse significant distances before encountering antigen.

Likewise the subsequent stage of detecting the presence or absence of the antibody-antigen combination may perhaps be speeded up for the reason given above and also because a large proportion of the available antibody in the urine sample has been concentrated in the membrane by virtue of the heavy loading with bacterial antigen. This may also facilitate washing steps between the stages of the assay.

It is also envisaged that the method of the invention may be applied by depositing different specific antigens or organisms within defined areas of a membrane and then adding to the membrane the urine sample under test. The area of the membrane in which a positive response is achieved could then be identified and an identification thus made of the nature of the antibodies in the sample. This may provide a rapid method of selecting the optimum form of treatment for the infection.

After incubation of the urine sample on the mixture of antigens the reaction mix should preferably be washed to remove uncombined excess, so that only the antibody in the antibody-antigen combination remains for detection. The detection of the antibody-antigen combination may be carried out by a variety of methods, for example by addition of an enzyme labelled reagent which combines with the antibody and can then be detected by colour change on addition of enzyme substrate. Antibody of IgG, IgM and IgA classes should be detected either in separate assays for example using specific anti-human immunoglobulins, or together using a polyvalent anti-human immunoglobulin. After addition of the enzyme labelled reagent, the reaction mix should again preferably be washed so that only reagent in combination with antibody remains for detection.

Embodiments of the present invention will now be described by way of illustration in the following Examples.

Example 1

Urine specimens were obtained from 85 patients with symptoms of urinary tract infection. For each patient, general practitioners provided details of symptoms and previous antibiotic therapy. Most patients had at least two of three symptoms - dysuria, frequency and urgency, and only three had clinical evidence of upper tract infection - loin pain, fever or rigors. Sequential samples were obtained from one patient, a six year old girl with symptoms of lower urinary tract infection. One patient had taken an antibiotic in the week before sample collection. Most specimens were processed on the day of collection, but no special arrangements were made for rapid transport of specimens to the laboratory. Catheter specimens were excluded from the study.

Control specimens were obtained from 40 healthy students (18 female, 22 male) who had no history of urinary tract infection during the previous four weeks.

Mid-stream urine specimens were collected in Boricon containers and cultured by inoculation on to MacConkey's agar with a Bacteriuritest filter strip according to the manufacturer's instructions. A pure and quantitatively significant growth of a lactose fermenting organism was recorded as >$10^5$ coliform bacilli/ml. Samples yielding >$10^5$ organisms/ml of a mixture of three or more organisms were discarded as contaminated, while samples yielding <$10^5$ organisms/ml were designated culture negative.

Microscopy was performed by examination of uncentrifuged urines at 400x magnification. Pyuria was defined as one or more polymorphs per 20 high power fields. Most urines from symptomatic patients were also screened for the presence of protein by Labstix and those showing trace protein or more were designated positive.

All samples were stored at 4°C while under investigation.

All the organisms used were cultured on nutrient agar for 48 hours at 37°C, except for Bacteroides fragilis which was cultured on blood agar anaerobically for 48 hours. The growth from two agar plates was harvested and suspended in 0.15M sterile saline and centrifuged at 3000g for 15 minutes. The pellet was re-suspended in 5ml 0.15M sterile saline and the concentration adjusted until a 1 in 10 dilution had an extinction of 0.25 at 540nm. This concentration was found in preliminary experiments to be optimal for antigen coating of plates. The suspension was then heated for 30 minutes at 100°C. Antigen preparations prepared in this way were used directly in absorption experiments, or mixed 9:1 (v/v) with 0.5M carbonate-bicarbonate buffer, pH9.6, and used to coat assay plates. Six coliform organisms were prepared as described and then mixed together in equal volumes before use in plate coating. Five of the organisms were identified by API 20E as E. Coli (3 strains), Klebsiella aerogenes, and Citrobacter freundii. The remaining organism was identified as Proteus mirabilis by routine biochemical tests. These organisms were selected arbitrarily from routine urine isolates to represent a range of common urinary pathogens. The three strains of E. Coli selected showed minor variations in their biochemical profiles on the API system.

Alkaline phosphatase labelled affinity purified anti-human IgG, IgA and IgM were diluted 1 in 400 in 0.05M phosphate buffered saline, pH7.4, to which 0.01% (v/v) Triton X-100 had been added (PBST). Anti-human anti-secretory component was conjugated to alkaline phosphatase by the method of Voller, Bidwell and Bartlett (Enzyme Immunoassays in Diagnostic Medicine. Theory and Practice. Bull WHO 1976; 53; 55-65). This was used in the antibody assay at a dilution of 1 in 100 in PBST.

Nunclon flat bottomed 96 Microwell plates were coated with 100ul of antigen preparation per well by incubation for 2 hours at 37°C and 16 hours at 4°C. Each well was washed three times with PBST and then incubated before use with 100ul of 1% (w/v) bovine serum albumin in 0.05M carbonate-bicarbonate buffer, pH9.6, for 2 hours at 37°C. After a further three washes, 100ul of each urine specimen was dispensed in duplicate and the plate incubated at 37°C for 1 hour. After washing, 100ul of anti-immunoglobulin conjugate was added to each well and the incubation and washing stages repeated. 100ul of a 1mg/ml solution of p-nitrophenyl phosphate in 0.05M glycine-NaOH buffer, pH10.2, was added to each well, and the colour in each well was measured 410nm after 30 minutes at room temperature. Each assay plate included a blank control well to which no urine was added, and also duplicate wells containing a reference positive sample.

The specificity of the antibody measured was investigated by pre-incubation of urine samples with an equal volume of mixed coliform antigen or other antigen preparations. The latter were prepared from single strains of Staphylococcus saprophyticus, Streptococcus faecalis, and Bacteroides fragilis. After 30 minutes incubation at 30°C, each mixture was assayed for IgG antibody to mixed coliform antigen as before.

The results of all antibody determinations were expressed as the average extinction at 410nm of duplicate results for each sample. The results were standardised against the values obtained for the reference positive sample in the appropriate assay run.

The significance of differences in antibody levels between groups was tested by the Mann-Whitney test, and of the different frequency of IgM in symptomatic groups by the $\chi^2$ test. The correlation coefficient r was calculated by standard methods.

The results are shown in Table 1.

Five samples were discarded as contaminated. Only 24 of the remaining 80 samples (30%) were culture positive, and all contained polymorphs. Culture negative samples were divided into those showing pyuria and those which did not. All control samples were negative on culture and microscopy.

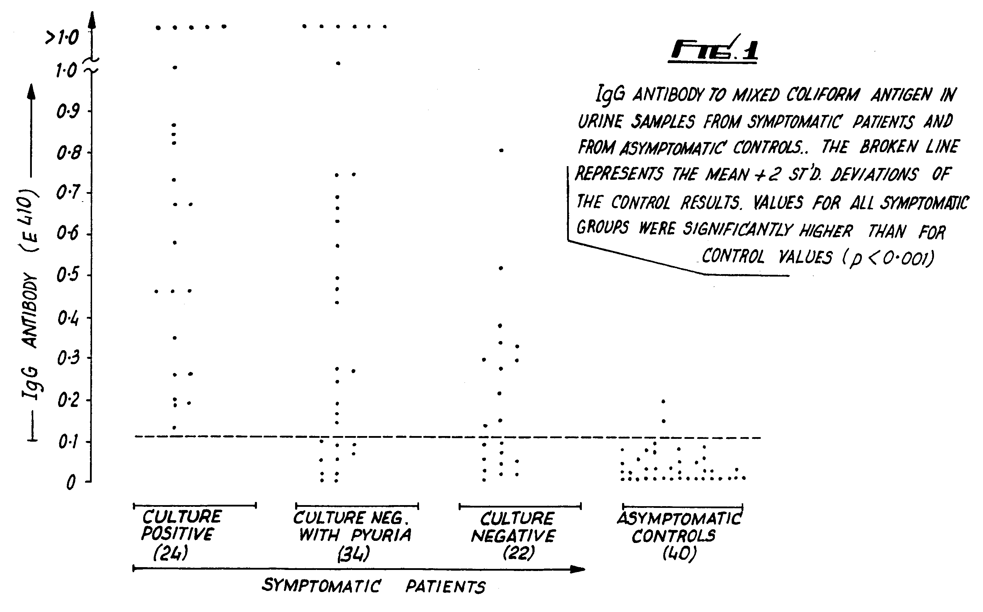

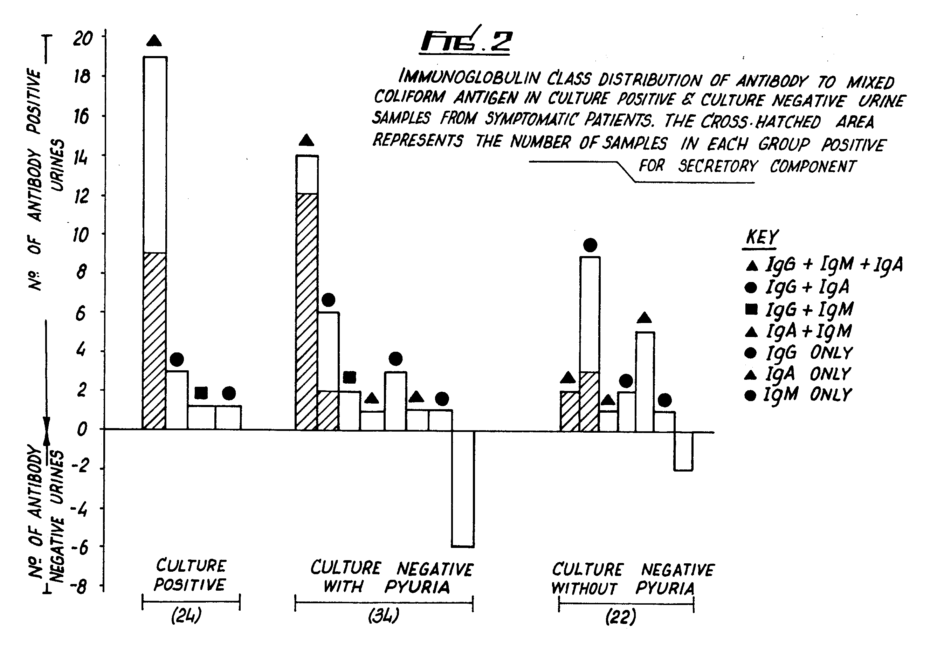

Antibody to the mixed coliform antigen was measured in all except the contaminated samples, and also in 40 urine samples from healthy volunteers. No significant differences were found between samples from male and female volunteers, and the control results were therefore pooled for further analysis. Figure 1 demonstrates that levels of IgG antibody to the mixed coliform antigen were significantly elevated in all symptomatic groups compared to asymptomatic controls (p<0.001). On the basis of similar results obtained for antibody in other immunoglobulin classes, samples were classified as positive for any one immunoglobulin class if they produced an ELISA reading greater than the calculated mean + 2 standard deviations for the control results. The resulting distribution of immunoglobulins in samples from different groups of symptomatic patients is shown in Figure 2. In total, 72 samples (90%) were positive for antibody in at least one immunoglobulin class, including all culture positive samples and 48 of the remaining 56 culture negative samples. The pattern of immunoglobulin distribution was not identical in all groups, however, with samples positive for all three immunoglobulins tested being predominant in the culture positive group and culture negative group with pyuria, while the combination of IgG and IgA was the commonest pattern in the culture negative group with no pyuria. The difference in the distribution of IgM among the three groups was statistically significant (p<0.01). The culture negative group with no pyuria also contained the largest number of specimens with IgA alone. Secretory component was detected in 35% of samples (28/80), and overall a significant correlation was found between IgA and secretory component (r = 0.63, p<0.01).

The specificity of the antibody measured was investigated by assay of selected urine samples following incubation of the urine with the mixed coliform antigen or with antigens prepared from other unrelated micro-organisms. The results in Table 2 show that the binding of IgG antibody to mixed coliform antigen was decreased only slightly by preincubation with antigens prepared by Staphylococcus saprophyticus, Streptococcus faecalis and Bacteroides fragilis, but was decreased significantly by pre-incubation with the mixed coliform antigen.

The spectrum of reactivity of urinary antibody to different coliform organisms was examined. Five culture positive specimens were tested for the presence of IgG antibody to each of the corresponding five coliform isolates. Considerable variation in the pattern of reactivity is shown in Table 3, and only one of the five specimens reacted maximally with its own isolate. The effectiveness of individual components of the mixed coliform antigen in binding antibody was studied by testing three culture positive and three culture negative urines against antigens prepared from the individual organisms. The results in Table 4 again show considerable variability in the pattern of reactivity of each urine specimen with different organisms. Most specimens reacted to some degree with each organism, but also showed a degree of specificity, in that each reacted maximally with one or two organisms. No single organism, however, was clearly more effective than the others at binding antibody.

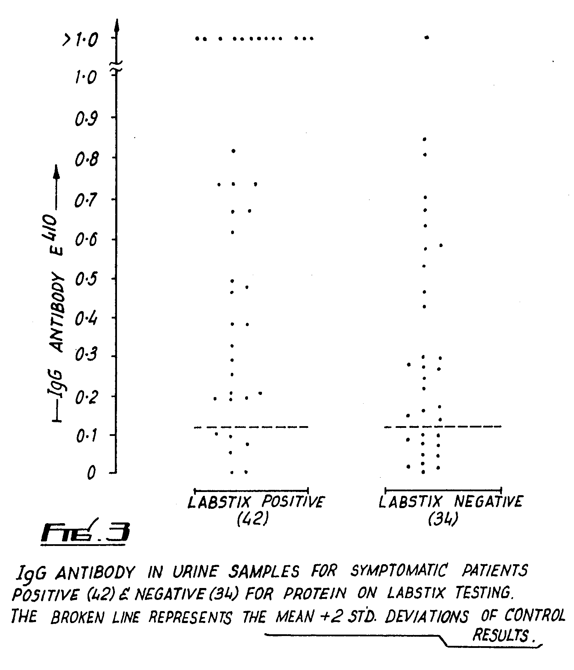

The sensitivity of the ELISA test was compared to that of dipstick testing for urinary protein using Labstix. Figure 3 shows that many ELISA positive urine samples were negative on dipstick testing for protein, and no significant difference was detected between IgG antibody levels in the Labstix positive and negative groups.

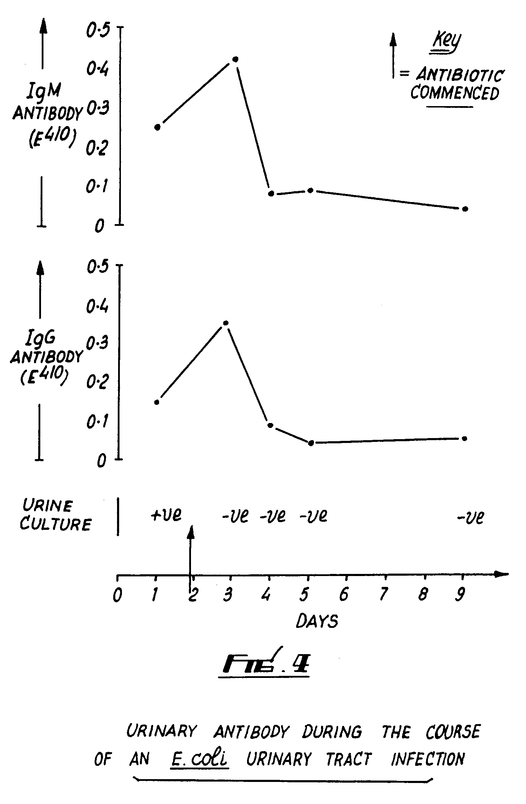

The time course of the urinary antibody response was investigated by testing sequential samples of urine from a six year old child with symptoms of lower urinary tract infection. No IgA or secretory component was detectable in any of the specimens, but the profile of IgG and IgM antibody detected is shown in Figure 4. The antibody persisted in urine for 24 hours after culture became negative, and then disappeared.

Antibody to a mixed coliform antigen was detected in a high proportion (90%) of urine samples from patients with symptoms of urinary tract infection, many of them culture negative by conventional criteria. The specificity of the antibody for coliform antigens was clearly demonstrated by the absorption experiments.

The components of the antigenic mixture used in this embodiment of the invention include O antigens as well as other such as H, K and fimbrial antigens.

Example 2

Plastics microtiter plates having 96 wells were coated with a mixture of the following organisms:

3 strains of Escherichia coli
Klebsiella species
Proteus mirabilis
Citrobacter freundii

The coating was performed by culture of the organisms on nutrient agar plates for 24 hours at 37°C, harvesting after incubation, suspending the organisms thus obtained in sterile saline, washing the organisms by centrifugation and re-suspension, heating the suspension at 100°C for 30 minutes, adjusting to optimum concentration by measurement of turbidity in a spectrophotometer, adding to 9 volumes of the suspension 1 volume of 1M carbonate/bicarbonate buffer to render the suspension alkaline to pH 9.6, and adding the suspension to the wells of the microtiter plates. The plates were allowed to sit for 2 hours at 37°C and then overnight at 4°C.

The plates were washed three times with 0.1M phosphate-buffered saline solution of pH 7.4 containing 0.1% Triton X-100 detergent, in order to remove unbound antigen. Empty sites remaining on the plates were then blocked by incubation of 1% human serum albumin (HSA) dissolved in 0.1M carbonate/bicarbonate buffer. Incubation was carried on for 2 hours at 37°C.

The plates were then washed again with 0.1M phosphate-buffered saline at pH 7.4 containing 0.1% Triton X-100. Urine samples to be tested for infection were then added one to each of the wells of the plates and incubated for 1 hour at 37°C. The samples in this case were undiluted, but in other Examples they could be diluted depending on their pH.

The plates were then washed as before, and enzyme-labelled antihuman immunoglobulin was added to bind to the antibody from the urine samples. The antihuman immunoglobulin was a 1 in 400 dilution of an alkaline phosphatase-linked commercial anti-immunoglobulin preparation from Sigma Chemical Company. Incubation was carried out for 1 hour at 37°C, and the plates were then washed as before.

A substrate consisting of a 1 mg per ml solution of paranitrophenol phosphate in 0.1M glycine sodium hydroxide buffer at pH 10.2 was added, and the plates were left to stand at room temperature for 30 minutes to allow a colour change to develop, demonstrating the presence of antibody in the original urine samples.

The method of this Example was performed on urine samples from 33 patients, all of whom displayed symptoms of urinary tract infection, and in 29 cases a positive result was obtained. In determining whether a result was positive or not, comparison was drawn with the same test performed on 40 patients who displayed no symptoms of infection, and the lower limit for a positive result was taken to be the level below which 95% of the test results on the asymptomatic patients fell.

In a comparative standard culture test performed on urine samples from the same 33 symptomatic patients, only 8 positive results were obtained.

The method illustrated in the above Examples has many advantages over the standard culture test previously used. For example:

1. the method allows positive differentiation to be made between urinary tract infection and other diseases whose symptoms may be similar, such as acute appendicitis; currently, such differentiation cannot be made with any certainty in view of the inaccuracy of the culture test;

2. the method allows further investigation into the cause of negative results in culture tests on symptomatic patients;

3. the method differentiates between the simple presence of organisms which are harmless contaminants, where no antibody is produced, and real infection in which antibody is created;

4. the method can be performed more quickly and cheaply than current culture tests, and produces a more accurate result.

It has been found that the patient's own isolate was less effective than other independently-provided organisms at binding antibody. It is possible that this is the result of the strain specific antibody in urine being absorbed by surface antigens present on the infecting organisms, with little remaining free for in vitro detection. There is also evidence that the infecting organism can change antigenically during the course of an infection, and the antibody present in urine may be directed against antigens which are no longer present when the organism is cultured. The rapid disappearance of antibody from urine during the course of a successfully treated infection is likely to provide a useful means of monitoring treatment by urinary antibody measurement. In patients with chronic recurrent symptoms or urinary tract infection, coliform organisms have been demonstrated in biopsy specimens of bladder mucosa despite failure to culture the organisms from urine. Urinary antibody may provide a marker of continuing infection in this group of patients, and is therefore useful in monitoring the effectiveness of antibiotic therapy.

The ELISA test described can be readily applied in the routine laboratory, and is suitable for automation. The test is a useful screening test for infection or a practical alternative to microscopy as an aid to the interpretation of culture results. The detection of urinary protein by dipstick testing is less sensitive and less specific since it is more likely to detect albumin than immunoglobulin.

EXAMPLE 3

Twenty-three organisms were prepared as described in Example 1 and total antibody in IgG, IgM and IgA immunoglobulin classes measured in 35 urine samples against each of these antigens. The organisms tested included 0 antigen strains of E. coli and Proteus which occur commonly in urinary tract infection, the organisms used in Example 1 for the mixture, and 5 strains of Pseudomonas isolated from urine samples. The results of optical density measurements (Table 5) show that some organisms are more effective than others at binding antibody, while the pattern of reactivity of individual urines across the range of organisms was extremely variable.

Four different antigen mixtures were prepared from the above 23 organisms. These comprised:

Mixture 1 - E. coli 01, 06, 09; Proteus 03; Pseudomonas 013R
Mixture 2 - E. coli 02, 04, 075; Proteus 027; Pseudomonas 846
Mixture 3 - As in Example 1 - 235, 418, 495, 916, 253, 500
Mixture 4 - E. coli 01, 09, 495, 916

Forty urine samples were tested for antibody in IgG, IgM and IgA immunoglobulin classes against each of these mixtures. The number of positive results (O.D. >0.1) obtained with each mixture were as follows:

Mixture 1 - 36
Mixture 2 - 31
Mixture 3 - 38
Mixture 4 - 39

The number of times a particular mixture gave the highest O.D. result for a particular urine sample in the test was as follows:

Mixture 1 - 7
Mixture 2 - 4
Mixture 3 - 6
Mixture 4 - 22

(one result unreadable in all 4).

Modifications and improvements may be made without departing from the scope of the invention.

TABLE 1

| Classification of urine specimens from 85 symptomatic patients by culture and microscopy | |
|---|---|
| Coliform culture positive | 24 |
| Culture negative with pyuria | 34 |
| Culture negative without pyuria | 22 |
| Contaminated specimens | 5 |
| | Total   85 |

7

TABLE 2

| IgG antibody ($E^{4\,10}$) to mixed coliform antigen in selected urine samples after pre-incubation with mixed coliform antigen or other unrelated antigens | | | | | |
|---|---|---|---|---|---|
| Urine | Antigen used for pre-incubation | | | | |
| | Control | *Mixed Coliform Antigen | Staphylococcus Saprophyticus | Streptococcus Faecalis | Bacteroides Fragilis |
| 1 | 1.03 | 0.21 | 0.97 | 0.98 | 1.03 |
| 2 | 0.36 | 0.03 | 0.38 | 0.34 | 0.34 |
| 3 | 1.76 | 0.51 | 1.59 | 1.55 | 1.58 |
| 4 | 1.36 | 0.15 | 1.28 | 0.98 | 1.23 |
| 5 | 0.51 | 0.06 | 0.44 | 0.39 | 0.39 |
| 6 | 0.42 | 0.04 | 0.39 | 0.41 | 0.39 |

*significantly lower than control values, $p < 0.05$

TABLE 3

| IgG antibody ($E^{4\,10}$) in 5 coliform culture positive urine samples to the corresponding 5 organisms | | | | | |
|---|---|---|---|---|---|
| Urine | Antigen preparation | | | | |
| | 1 | 2 | 3 | 4 | 5 |
| 1 | 0.13 | 1.21 | 0.55 | 0.33 | 0.86 |
| 2 | 0.23 | 0.36 | 0.18 | 0.20 | 0.27 |
| 3 | 0.48 | 0.70 | 0.49 | 0.33 | 1.19 |
| 4 | 0.59 | 0.99 | 0.22 | 0.37 | 0.26 |
| 5 | 0.31 | 0.67 | 0.48 | 0.43 | 0.44 |

## TABLE 4

IgG antibody ($E^{410}$) to separate components of the mixed coliform antigen in 3 culture positions and 3 culture negative urine samples

Antigen Preparation

| Urine | Culture Result | E. coli (1) | E. coli (2) | E. coli (3) | Klebsiella aerogenes | Citrobacter freundii | Proteus mirabilis |
|---|---|---|---|---|---|---|---|
| 1 | $10^5$ coliform bacilli/ml | 0.26 | 0.11 | 0.19 | 0.18 | 0.97 | 0.26 |
| 2 | $10^5$ coliform bacilli/ml | 0.51 | 0.99 | 1.45 | >1.99 | 1.46 | 0.22 |
| 3 | $10^5$ coliform bacilli/ml | 0.25 | 1.58 | 0.22 | 0.25 | 0.19 | 0.18 |
| 4 | negative | >1.99 | 1.59 | 0.38 | 0.35 | 0.52 | 0.24 |
| 5 | negative | 1.33 | 0.14 | 0.31 | 0.31 | 0.26 | 0.18 |
| 6 | negative | 0.19 | 1.15 | 0.12 | 0.33 | 0.57 | 0.10 |

EP 0 233 048 B1

TABLE 5

| Organism | E. Coli | | | | | | |
|---|---|---|---|---|---|---|---|
| O Antigen or lab No.<br>Urine No. | 01 | 02 | 04 | 05 | 06 | 09 | 075 |
| 13587 | 0.061 | 0.040 | 0.041 | 0.075 | 0.230 | 0.070 | 0.087 |
| 14116 | 0.142 | 0.054 | 0.123 | 0.116 | 0.065 | 0.592 | 0.116 |
| 14175 | 0.149 | 0.085 | 0.064 | 0.089 | 0.068 | 0.119 | 0.079 |
| 14233 | 0.101 | 0.017 | 0.061 | 0.07 | 0.10 | 0.106 | 0.083 |
| 14312 | 0.543 | 0.223 | 0.411 | 0.616 | 0.539 | 0.992 | 0.429 |
| 14356 | 0.127 | 0.048 | 0.064 | 0.061 | 0.041 | 0.052 | 0.046 |
| 14560 | 0.08 | 0.04 | 0.111 | 0.079 | 0.035 | 0.078 | 0.159 |
| 13176 | 0.152 | 0.081 | 0.084 | 0.159 | 0.115 | 0.14 | 0.09 |
| 14107 | 0.146 | 0.054 | 0.094 | 0.206 | 0.165 | 0.066 | 0.099 |
| 14134 | 0.056 | 0.065 | 0.102 | 0.085 | 0.047 | 0.112 | 0.053 |
| 14238 | 0.088 | 0.063 | 0.035 | 0.037 | 0.03 | 0.051 | 0.05 |
| 14608 | 0.135 | 0.06 | 0.046 | 0.062 | 0.055 | 0.078 | 0.278 |
| 15614 | 0.077 | 0.035 | 0.642 | 0.071 | 0.036 | 0.107 | 0.063 |
| 15713 | 0.035 | 0.02 | 0.02 | 0.022 | 0.024 | 0.036 | 0.02 |
| 14836 | 0.08 | 0.041 | 0.039 | 0.058 | 0.281 | 0.589 | 0.068 |
| 14988 | 0.077 | 0.177 | 0.07 | 0.103 | 0.04 | 0.113 | 0.053 |
| 15161 | 0.313 | 0.062 | 0.079 | 0.249 | 0.141 | 0.201 | 0.196 |
| 15163 | 0.072 | 0.052 | 0.033 | 0.055 | 0.391 | 0.063 | 0.046 |
| 15175 | 0.094 | 0.04 | 0.068 | 0.069 | 0.604 | 0.42 | 0.057 |
| 15365 | 0.135 | 0.066 | 0.153 | 0.149 | 0.205 | 0.175 | 0.1 |
| 15599 | 0.011 | 0.026 | 0.026 | 0.03 | 0.024 | 0.028 | 0.024 |
| 15835 | 0.144 | 0.072 | 0.053 | 0.084 | 0.028 | 0.012 | 0.042 |
| 15928 | 0.559 | 0.024 | 0.019 | 0.316 | 0.039 | 0.172 | 1.677 |
| 15943 | 0.178 | 0.124 | 0.073 | 0.035 | 0.020 | 0.100 | 0.042 |
| 16033 | 0.017 | 0.021 | 0.001 | 0.004 | 0.071 | 0.003 | 0.004 |
| 16172 | 0.149 | 0.099 | 0.008 | 0.139 | 0.108 | 0.125 | 0.104 |
| 16271 | 0.005 | 0.07 | 0.022 | 0.040 | 0.003 | 0.052 | 0.013 |
| 16290 | 0.177 | 0.030 | 0.084 | 0.035 | 0.142 | 0.030 | 0.082 |
| 16491 | 0.034 | 0.042 | 0.001 | 0.165 | 0.005 | 0.009 | 0.001 |
| 16493 | 0.276 | 0.232 | 0.023 | 0.165 | 0.065 | 0.205 | 0.413 |
| 16496 | 0.145 | 0.309 | 0.134 | 0.178 | 0.050 | 0.270 | 0.065 |
| 16596 | 0.132 | 0.077 | 0.001 | 0.109 | 0.073 | 0.564 | 0.432 |
| 16638 | 0.006 | 0.007 | 0.004 | 0.030 | 0.004 | 0.002 | 0.409 |
| 16673 | 0.142 | 0.001 | 0.008 | 0.082 | 0.011 | 0.113 | 0.055 |
| 16730 | 1.586 | 1.732 | 1.688 | OVER | 0.701 | 1.530 | OVER |
| No. of Positives (≥ 0.10) | 20 | 6 | 7 | 14 | 13 | 20 | 12 |

TABLE 5
(Cont.1)

| Organism | Proteus | | | | Kleb-siella | E. Coli | | |
|---|---|---|---|---|---|---|---|---|
| O Antigen or lab No. / Urine No. | 03 | 010 | 021 | 028 | 235 | 418 | 495 | 916 |
| 13587 | 0.088 | 0.047 | 0.095 | 0.053 | 0.183 | 0.097 | 0.140 | 0.231 |
| 14116 | 0.100 | 0.111 | 0.072 | 0.106 | 0.175 | 0.072 | 0.107 | 0.072 |
| 14175 | 0.07 | 0.082 | 0.049 | 0.083 | 0.094 | 0.102 | 0.083 | 0.082 |
| 14233 | 0.033 | 0.036 | 0.028 | 0.031 | 0.099 | 0.131 | 0.077 | 0.102 |
| 14312 | 0.565 | 0.680 | 0.375 | 0.492 | 0.617 | 0.351 | 0.462 | 0.543 |
| 14356 | 0.025 | 0.028 | 0.021 | 0.035 | 0.071 | 0.059 | 0.031 | 0.103 |
| 14560 | 0.11 | 0.077 | 0.053 | 0.07 | 0.126 | 0.059 | 0.129 | 0.045 |
| 13176 | 0.273 | 0.335 | 0.134 | 0.268 | 0.282 | 0.136 | 0.12 | 0.134 |
| 14107 | 0.088 | 0.093 | 0.062 | 0.083 | 0.123 | 0.183 | 0.105 | 0.128 |
| 14134 | 0.038 | 0.038 | 0.025 | 0.032 | 0.281 | 0.084 | 0.052 | 0.047 |
| 14238 | 0.03 | 0.031 | 0.027 | 0.024 | 0.102 | 0.058 | 0.049 | 0.031 |
| 14608 | 0.057 | 0.062 | 0.044 | 0.051 | 0.123 | 0.100 | 0.257 | 0.058 |
| 15614 | 0.043 | 0.046 | 0.029 | 0.045 | 0.07 | 0.088 | 0.049 | 0.039 |
| 15713 | 0.02 | 0.022 | 0.016 | 0.022 | 0.026 | 0.024 | 0.025 | 0.023 |
| 14836 | 0.068 | 0.068 | 0.054 | 0.057 | 0.078 | 0.053 | 0.062 | 0.196 |
| 14928 | 0.054 | 0.052 | 0.041 | 0.051 | 0.063 | 0.21 | 0.034 | 0.052 |
| 15161 | 0.312 | 0.319 | 0.205 | 0.204 | 0.581 | 0.148 | 0.238 | 0.130 |
| 15163 | 0.057 | 0.058 | 0.053 | 0.063 | 0.06 | 0.048 | 0.054 | 0.267 |
| 15175 | 0.044 | 0.081 | 0.057 | 0.092 | 0.121 | 0.045 | 0.067 | 0.363 |
| 15365 | 0.112 | 0.089 | 0.064 | 0.092 | 0.136 | 0.09 | 0.099 | 0.131 |
| 15599 | 0.03 | 0.028 | 0.025 | 0.001 | 0.037 | 0.027 | 0.034 | 0.021 |
| 15835 | 0.007 | 0.012 | 0.029 | 0.031 | 0.040 | 0.068 | 0.021 | 0.156 |
| 15928 | 0.165 | 0.260 | 0.014 | 0.054 | 0.154 | 0.112 | 1.145 | 0.046 |
| 15943 | 0.041 | 0.041 | 0.065 | 0.044 | 0.075 | 0.033 | 0.036 | 0.008 |
| 16033 | 0.037 | 0.026 | 0.046 | 0.020 | 0.009 | 0.006 | 0.021 | 0.063 |
| 16172 | 0.162 | 0.207 | 0.071 | 0.259 | 0.262 | 0.142 | 0.152 | 0.165 |
| 16271 | 0.004 | 0.003 | 0.001 | 0.060 | 0.032 | 0.010 | 0.006 | 0.052 |
| 16290 | 0.042 | 0.024 | 0.020 | 0.017 | 0.032 | 0.007 | 0.051 | 0.182 |
| 16491 | 0.034 | 0.056 | 0.001 | 0.038 | 0.085 | 0.027 | 0.068 | 0.027 |
| 16493 | 0.174 | 0.282 | 0.111 | 0.228 | 0.262 | 0.136 | 0.356 | 0.062 |
| 16496 | 0.065 | 0.105 | 0.061 | 0.053 | 0.175 | 0.475 | 0.101 | 0.142 |
| 16596 | 0.026 | 0.052 | 0.004 | 0.078 | 0.076 | 0.017 | 0.267 | 0.122 |
| 16638 | 0.002 | 0.001 | 0.020 | 0.015 | 0.021 | 0.008 | 0.265 | 0.009 |
| 16673 | 0.003 | 0.021 | 0.014 | 0.019 | 0.030 | 0.015 | 0.020 | 0.007 |
| 16730 | 0.858 | 1.120 | 0.594 | 0.923 | OVER | 1.299 | 1.524 | 0.867 |
| No. of Positives (≥ 0.10) | 10 | 9 | 5 | 7 | 17 | 13 | 15 | 17 |

| Organism | Citro-Bacter | Proteus | Pseudomonas | | | | |
|---|---|---|---|---|---|---|---|
| O Antigen or lab No. | 253 | 500 | 284 9EN | 013R | 946 | 795B | 846 |
| Urine No. | | | | | | | |
| 13587 | 0.150 | 0.067 | 0.034 | 0.035 | 0.047 | 0.031 | 0.024 |
| 14116 | 0.175 | 0.101 | 0.181 | 0.215 | 0.173 | 0.083 | 0.143 |
| 14175 | 0.06 | 0.07 | 0.071 | 0.103 | 0.168 | 0.171 | 0.057 |
| 14233 | 0.051 | 0.031 | 0.024 | 0.06 | 0.045 | 0.029 | 0.023 |
| 14312 | 0.525 | 0.507 | 0.354 | 0.431 | 0.436 | 0.381 | 0.349 |
| 14356 | 0.277 | 0.029 | 0.036 | 0.034 | 0.035 | 0.037 | 0.029 |
| 14560 | 0.067 | 0.063 | 0.046 | 0.065 | 0.045 | 0.056 | 0.036 |
| 13176 | 0.148 | 0.304 | 0.066 | 0.072 | 0.049 | 0.043 | 0.043 |
| 14107 | 0.088 | 0.089 | 0.073 | 0.073 | 0.086 | 0.099 | 0.041 |
| 14134 | 0.101 | 0.031 | 0.055 | 0.097 | 0.105 | 0.051 | 0.048 |
| 14238 | 0.067 | 0.034 | 0.047 | 0.044 | 0.037 | 0.026 | 0.026 |
| 14608 | 0.071 | 0.059 | 0.076 | 0.145 | 0.051 | 0.044 | 0.064 |
| 15614 | 0.054 | 0.054 | 0.062 | 0.064 | 0.048 | 0.062 | 0.04 |
| 15713 | 0.022 | 0.023 | 0.023 | 0.022 | 0.025 | 0.026 | 0.02 |
| 14836 | 0.056 | 0.056 | 0.038 | 0.052 | 0.041 | 0.042 | 0.036 |
| 14998 | 0.058 | 0.047 | 0.04 | 0.047 | 0.044 | 0.054 | 0.035 |
| 15161 | 0.223 | 0.246 | 0.037 | 0.035 | 0.03 | 0.038 | 0.036 |
| 15163 | 0.059 | 0.068 | 0.052 | 0.047 | 0.031 | 0.052 | 0.034 |
| 15175 | 0.077 | 0.094 | 0.202 | 0.061 | 0.071 | 0.167 | 0.04 |
| 15365 | 0.131 | 0.083 | 0.166 | 0.294 | 0.162 | 0.22 | 0.145 |
| 15599 | 0.028 | 0.032 | 0.04 | 0.036 | 0.027 | 0.038 | 0.025 |
| 15835 | 0.014 | 0.031 | 0.001 | 0.005 | 0.006 | 0.020 | 0.008 |
| 15928 | 0.058 | 0.099 | 0.003 | 0.003 | 0.037 | 0.174 | 0.010 |
| 15943 | 0.053 | 0.051 | 0.002 | 0.007 | 0.001 | 0.030 | 0.006 |
| 16033 | 0.061 | 0.003 | 0.014 | 0.042 | 0.008 | 0.156 | 0.004 |
| 16172 | 0.185 | 0.277 | 0.059 | 0.021 | 0.051 | 0.302 | 0.005 |
| 16271 | 0.007 | 0.006 | 0.059 | 0.019 | 0.067 | 0.007 | 0.069 |
| 16290 | 0.001 | 0.006 | 0.016 | 0.020 | 0.058 | 0.249 | 0.06 |
| 16491 | 0.010 | 0.066 | 0.010 | 0.044 | 0.020 | 0.064 | 0.001 |
| 16493 | 0.091 | 0.235 | 0.074 | 0.241 | 0.001 | 0.093 | 0.004 |
| 16496 | 0.158 | 0.076 | 0.201 | 0.202 | 0.143 | 0.016 | 0.103 |
| 16596 | 0.002 | 0.121 | 0.006 | 0.183 | 0.001 | 0.257 | 0.008 |
| 16638 | 0.002 | 0.017 | 0.002 | 0.002 | 0.004 | 0.006 | 0.001 |
| 16673 | 0.002 | 0.039 | 0.030 | 0.115 | 0.008 | 0.047 | 0.003 |
| 16730 | 1.302 | 0.832 | 0.807 | 1.612 | 0.788 | 1.196 | 0.392 |
| No. of Positives (≥ 0.10) | 11 | 8 | 6 | 10 | 7 | 10 | 5 |

TABLE 5 (Cont.2)

## Claims

1. A method of detecting urinary tract infection or inflammation comprising providing a urine sample from a patient,

EP 0 233 048 B1

adding to said urine sample a mixture of antigens prepared from a mixture of organisms which are known to occur in urinary tract infection by killing said organisms without substantial reduction of the antigen content of the organisms, said organisms being obtained independently of said patient's urine,

and detecting the presence or absence of a combination of antibody from said urine sample with antigen from said mixture of antigens.

2. A method according to Claim 1, wherein said mixture of organisms is killed by heating or by sonicating the organisms to an extent insufficient to reduce substantially the antigen content of the organisms.

3. A method according to Claim 2, wherein said organisms are killed by heating at 100ºC for 30 minutes.

4. A method according to any one of the preceding Claims, wherein said organisms are Gram negative.

5. A method according to Claim 4, wherein at least one of said organisms is coliform.

6. A method according to Claim 5, wherein at least one of said organisms is Escherichia coli.

7. A method according to Claim 6, wherein said mixture of antigens comprises antigens from Escherichia coli, Klebsiella aerogenes, Citrobacter freundii and Proteus mirabilis.

8. A method according to Claims 1, 2 or 3, wherein said mixture of antigens is prepared from Gram positive organisms.

9. A method according to Claim 8, wherein said mixture of antigens includes Staphylococcal antigens.

10. A method according to any one of the preceding Claims, wherein the presence or absence of said combination of antibody and antigen is detected by washing said mixture of antigens after addition of said urine sample and adding to the washed mixture a labelled reagent which is known to combine with antibody which combines with said antigens, washing excess of said reagent from said mixture and thereafter detecting the presence or absence of said label on the mixture.

**Patentansprüche**

1. Verfahren zum Nachweis einer Infektion oder Entzündung des Harntraktes, bei welchem

eine Urinprobe von einem Patienten bereitgestellt wird,
der Urinprobe ein Gemisch eines Antigens hinzugefügt wird, das aus einem Gemisch von Organismen zubereitet wurde, die für ein Vorkommen bei einer Infektion des Harntraktes bekannt sind, wobei die Organismen ohne eine wesentliche Verringerung des Antigengehalts der Organismen getötet werden und die Organismen unabhängig von dem Urin des Patienten erhalten sind,
und die Anwesenheit oder Abwesenheit einer Kombination eines Antikörpers bei der Urinprobe mit einem Antigen dieses Antigengemisches nachgewiesen wird.

2. Verfahren nach Anspruch 1, bei welchem das Gemisch der Organismen durch ein Erhitzen oder durch eine Schallbeaufschlagung der Organismen in einem Ausmaß getötet wird, welches nicht ausreicht, den Antigengehalt der Organismen wesentlich zu verringern.

3. Verfahren nach Anspruch 2, bei welchem die Organismen durch ein Erhitzen bei 100°C für 30 Minuten getötet werden.

4. Verfahren nach einem der vorhergehenden Ansprüche, bei welchem die Organismen Gram negativ sind.

5. Verfahren nach Anspruch 4, bei welchem wenigstens einer der Organismen coliform ist.

6. Verfahren nach Anspruch 5, bei welchem wenigstens einer der Organismen Escherichia coli ist.

7. Verfahren nach Anspruch 6, bei welchem das Antigengemisch Antigene von Escherichia coli, Klebsiella

13

aerogenes, Citrobacter freundii und Proteus mirabilis aufweist.

8. Verfahren nach den Ansprüchen 1, 2 oder 3, bei welchem das Antigengemisch von Gram Positiv-Organismen zubereitet wird.

9. Verfahren nach Anspruch 8, bei welchem das Antigengemisch Staphylococcal-Antigene enthält.

10. Verfahren nach einem der vorhergehenden Ansprüche, bei welchem die Anwesenheit oder Abwesenheit der Kombination eines Antikörpers und eines Antigens durch ein Auswaschen des Antigengemisches nach der Hinzufügung der Urinprobe und der Hinzufügung eines beschrifteten Reagenz zu dem ausgewaschenen Gemisch nachgewiesen wird, das für eine Kombination mit einem Antikörper bekannt ist und sich mit dem Antigen kombiniert, ein Überschuß des Reagenz aus dem Gemisch ausgewaschen wird und danach die Anwesenheit oder Abwesenheit des beschrifteten Reagenz ermittelt wird.

**Revendications**

1. Procédé pour la détection d'infection ou inflammation du faisceau urinaire, qui consiste :
   - à prélever un échantillon d'urine d'un patient ;
   - à ajouter audit échantillon un mélange d'antigènes préparé à partir d'un mélange d'organismes obtenus indépendamment de l'urine du patient, et qui sont connus pour se produire dans une infection du faisceau urinaire, en tuant lesdits organismes sans réduction substantielle des antigènes contenus dans lesdits organismes ;
   - et à détecter la présence ou l'absence d'une combinaison des anticorps de l'échantillon d'urine avec des antigènes dudit mélange d'antigènes.

2. Procédé selon la revendication 1, dans lequel ledit mélange d'organismes est tué par chauffage ou par voie accoustique des organismes à un niveau insuffisant pour réduire substantiellement la teneur en antigène desdits organismes.

3. Procédé selon la revendication 2, dans lequel les organismes sont tués par chauffage à 100°C pendant trente minutes.

4. Procédé selon l'une des revendications précédentes, dans lequel les organismes sont des Gram négatifs.

5. Procédé selon la revendication 4, dans lequel au moins un des organismes est coliforme.

6. Procédé selon la revendication 5, dans lequel au moins un des organismes est l'Escherichia coli.

7. Procédé selon la revendication 6, dans lequel le mélange d'antigènes comprend des antigènes de Escherichia coli, Klebsiella aerogenes, Citrobacter freundii et Proteus mirabilis.

8. Procédé selon l'une des revendications 1,2 ou 3, dans lequel le mélange d'antigènes est préparé à partir d'organismes Gram positifs.

9. Procédé selon la revendication 8, dans lequel ledit mélange d'antigènes comprend des antigènes de Staphylocoque.

10. Procédé selon l'une des revendications précédentes, dans lequel la présence ou l'absence de ladite combinaison d'anticorps et d'antigènes est détectée par lavage desdits mélanges d'antigènes après addition de l'échantillon d'urine, puis addition à ce mélange de lavage d'un réactif identifié qui est connu pour se combiner avec des anticorps qui se combinent avec lesdits antigènes, puis lavage de l'excès dudit réactif du mélange, et ensuite détection de la présence ou l'absence dudit réactif sur le mélange.

**FIG. 1**

IgG ANTIBODY TO MIXED COLIFORM ANTIGEN IN URINE SAMPLES FROM SYMPTOMATIC PATIENTS AND FROM ASYMPTOMATIC CONTROLS.. THE BROKEN LINE REPRESENTS THE MEAN +2 ST'D. DEVIATIONS OF THE CONTROL RESULTS. VALUES FOR ALL SYMPTOMATIC GROUPS WERE SIGNIFICANTLY HIGHER THAN FOR CONTROL VALUES ($p < 0.001$)

EP 0 233 048 B1

FIG.2

IMMUNOGLOBULIN CLASS DISTRIBUTION OF ANTIBODY TO MIXED COLIFORM ANTIGEN IN CULTURE POSITIVE & CULTURE NEGATIVE URINE SAMPLES FROM SYMPTOMATIC PATIENTS. THE CROSS-HATCHED AREA REPRESENTS THE NUMBER OF SAMPLES IN EACH GROUP POSITIVE FOR SECRETORY COMPONENT

KEY
▲ IgG + IgM + IgA
● IgG + IgA
■ IgG + IgM
▲ IgA + IgM
● IgG ONLY
▲ IgA ONLY
● IgM ONLY

EP 0 233 048 B1

FIG.3

IgG ANTIBODY IN URINE SAMPLES FOR SYMPTOMATIC PATIENTS POSITIVE (42) & NEGATIVE (34) FOR PROTEIN ON LABSTIX TESTING. THE BROKEN LINE REPRESENTS THE MEAN +2 STD. DEVIATIONS OF CONTROL RESULTS.

17

**Fig. 4**

URINARY ANTIBODY DURING THE COURSE OF AN *E. coli* URINARY TRACT INFECTION